# EUROPÄISCHE PATENTSCHRIFT

(11) **EP 1 147 782 B1**
(45) Veröffentlichungstag und Bekanntmachung des Hinweises auf die Patenterteilung: **11.01.2006**
(21) Anmeldenummer: 01105434.3
(22) Anmeldetag: 13.03.2001
(51) Int. Cl.: A61M 16/06

(54) **Atemmaskenanordnung**
Breathing mask
Masque respiratoire

(30) Priorität: 18.04.2000 DE 10019358
(43) Veröffentlichungstag der Anmeldung: 24.10.2001
(73) Patentinhaber: Map-Medizintechnologie GmbH, 82152 Martinsried (DE)
(72) Erfinder: Biener, Achim, 80636 München (DE); Lang, Bernd, 82166 Gräfelfing (DE)
(74) Vertreter: Vossius & Partner

(56) Entgegenhaltungen:
- EP-A- 0 462 701
- WO-A-00/20072
- DE-A- 3 935 890
- DE-A- 19 721 279
- DE-A- 19 807 961

## Beschreibung

Die Erfindung betrifft eine Atemmaskenanordnung zur Zufuhr eines Atemgases unter Überdruck. Derartige Atemmaskenanordnungen finden insbesondere im Bereich der Schlafmedizin zur Behandlung schlafbezogener Atmungsstörungen Anwendung. Eine Atemmaskenanordnung gemäß Oberbegriff von Patentanpruch 1 ist z.B. in WO-A-00/20072 beschrieben.

Bei den bekannten Atemmaskenanordnungen ist üblicherweise ein Maskenbasiskörper vorgesehen, der aus einer Hartschale oder auch aus einem Elastomermaterial gebildet sein kann, und welcher üblicherweise mit einer Schlauchanschlusseinrichtung versehen ist zum Anschluss eines Atemschlauches. Diese Atemmaskenanordnung wird üblicherweise durch ein Kopfband am Kopf des Maskenanwenders fixiert. Die Abdichtung des durch den Maskenbasiskörper begrenzten Maskeninnenbereichs zur Umgebung hin erfolgt üblicherweise über eine Dichtlippeneinrichtung einer hochflexiblen Dichtungsanordnung.

Um einen möglichst hohen Tragekomfort der Atemmaske zu erreichen, wird der Anpressdruck der Dichtlippeneinrichtung gegen das Gesicht des Patienten möglichst gering gehalten und auch die Dichtlippeneinrichtung selbst möglichst dünnwandig ausgebildet, so dass die seitens der Dichtlippeneinrichtung auf die Gesichtsfläche des Patienten ausgeübte Flächenpressung keine unzulässig hohen Werte erreicht.

Es hat sich jedoch gezeigt, dass selbst Dichtlippeneinrichtungen, bei welchen die geforderte Dichtwirkung unter vergleichsweise geringen Flächenpressungen erreicht werden kann, nach einer längeren Einsatzdauer Unannehmlichkeiten bereiten.

Der Erfindung liegt die Aufgabe zugrunde, diese mit der Langzeitanwendung der Atemmasken verbundenen Unannehmlichkeiten auszuräumen oder zumindest abzumildern.

Diese Aufgabe wird erfindungsgemäß gelöst durch eine Atemmaskenanordnung mit einem Maskenbasiskörper, einer Schlauchanschlusseinrichtung zum Anschluss eines Atemschlauches, einer Befestigungseinrichtung zur Fixierung des Maskenbasiskörpers im Nasenbereich eines Maskenanwenders und einer Dichtungsanordnung zur Abdichtung eines durch den Maskenbasiskörper begrenzten Maskeninnenraums gegenüber der Umgebung, wobei die Dichtungsanordnung eine aus einem elastomeren Material gebildete Dichtlippeneinrichtung aufweist, die entlang einer Gesichtsäuflagezone unter einer definierten Flächenpressungsverteilung auf dem Gesicht des Maskenanwenders aufliegt, wobei die Dichtlippeneinrichtung aus einem Set aus wenigstens zwei unterschiedlichen Dichtlippeneinrichtungen ausgewählt ist und die Dichtlippeneinrichtungen des Sets sich hinsichtlich der Flächenpressungsverteilung und/oder dem Verlauf der Gesichtsauflagezone unterscheiden und für den Maskenanwender jeweils passen.

Dadurch wird es auf vorteilhafte Weise möglich, in vorgegebenen Zeitabständen, beispielsweise jeden zweiten Tag, die Dichtlippeneinrichtung zu wechseln und hierbei die Regeneration der durch die Maskenanwendung belasteten Gesichtszonen zu unterstützen.

Die einzelnen Dichtlippeneinrichtungen sind vorzugsweise derart aufeinander abgestimmt, dass die Atemmaskenanordnung unmittelbar nach Austausch der Dichtlippeneinrichtung gebrauchsfertig ist, ohne dass hierbei erheblicher Nachjustierungsbedarf besteht.

Eine besonders bevorzugte Ausführungsform der Erfindung ist dadurch gegeben, dass die Dichtlippeneinrichtungen Befestigungsstrukturen aufweisen, die zu einer maskenbasiskörperseitigen Befestigungsstruktur kompatibel sind. Hierdurch wird es auf kostengünstige Weise möglich, an ein und demselben Maskenbasiskörper unterschiedliche, jedoch für den Maskenanwender in gleicher Weise passende Dichtlippeneinrichtungen zu fixieren.

In vorteilhafter Weise werden diese unterschiedlichen Dichtlippeneinrichtungen aus einem Dichtlippenset ausgewählt, das wenigstens drei unterschiedliche Dichtlippeneinrichtungen aufweist.

Die Dichtlippeneinrichtungen können entweder derart vorgefertigt sein, dass diese von sich aus unterschiedliche Dichtlippenauflagezonen, bzw. unterschiedliche Flächenpressungen herbeiführen. Alternativ dazu ist es jedoch auch möglich, die unterschiedlichen Dichtlippeneinrichtungen dadurch bereitzustellen, dass in ihrem Aufbau identische Dichtlippeneinrichtungen in unterschiedlicher Weise für den Maskenanwender individuell passend ausgeformt werden. Dies kann beispielsweise dadurch erfolgen, das in die Dichtlippeneinrichtung ein aus einem plastisch verformbaren Material, beispielsweise Metall, gefertigtes Verstärkungselement eingefügt ist, das der Dichtlippeneinrichtung einen individuell passend angeformten Verlauf verleiht.

In vorteilhafter Weise umfasst wenigstens eine der Dichtlippeneinrichtungen einen Gelkörper. Dadurch wird es möglich, im täglichen oder wöchentlichen Wechsel Geldichtkissen mit reinen Dichtlippendichtkissen auszutauschen.

Gem. einer besonders bevorzugten Ausführungsform der Erfindung sind die jeweiligen Dichtlippeneinrichtungen des Dichtlippensets derart gebildet, dass diese insbesondere hinsichtlich des Verlaufs im Nasenrückenbereich unterschiedliche Dichtlippenauflagezonen definieren. Alternativ dazu oder auch in Kombination mit dieser Maßnahme ist es auch möglich, die Dichtlippeneinrichtungen derart zu wählen, dass diese insbesondere in dem, den Nasenflügeln benachbarten Bereich des Maskenanwenders unterschiedliche Dichtlippenauflagezonen definieren.

Gem. einer besonders bevorzugten Ausführungsform der Erfindung ist jede Dichtlippeneinrichtung mit einem Rahmenelement versehen zur definierten Befestigung der Dichtlippeneinrichtung an einem entsprechend komplementär ausgebildeten Maskenbasiskörper. Vorzugsweise wird dieses Rahmenelement über einen Klemmsitz oder über eine Schnappverbindungseinrichtung in abdichtender Weise an dem Maskenbasiskörper fixiert.

Alternativ zu einem Austausch lediglich der Dichtlippeneinrichtung ist es auch möglich, unterschiedliche Einheiten von Maskenbasiskörper und Dichtlippeneinrichtung bereitzustellen, die insgesamt austauschbar sind. Die wechselweise Integration der jeweiligen Einheiten aus Maskenbasiskörper und Dichtlippeneinrichtung erfolgt über eine entsprechende Anschlussstruktur zwischen der Atemschlauchanschlusseinrichtung und dem Maskenbasiskörper.

Weitere vorteilhafte Ausgestaltungen der Erfindung sind Gegenstand der Unteransprüche.

Weitere Einzelheiten ergeben sich aus der nachfolgenden Beschreibung mehrerer bevorzugter Ausführungsbeispiele in Verbindung mit der Zeichnung. Es zeigt:
**Fig. 1a:** eine vereinfachte Seitenansicht eines Maskenbasiskörpers mit einer zu drei unterschiedlichen Dichtungsanordnungen kompatiblen Verbindungsstruktur;
**Fig. 1b:** eine vereinfachte Seitenansicht einer ersten Ausführungsform einer zum Maskenbasiskörper nach Fig. 1a kompatiblen Dichtkisseneinrichtung, die sich primär in dem die Oberlippe überquerenden Bereich sowie im Bereich der Nasenflügel auf dem Gesicht des Patienten abstützt;
**Fig. 1c:** eine ebenfalls zum Maskenbasiskörper nach Fig. 1a kompatible Dichtlippeneinrichtung, die sich überwiegend im Nasenseitenbereich des Maskenanwenders abstützt;
**Fig. 1d:** eine ebenfalls vereinfachte Seitenansicht einer weiteren Ausführungsform einer zum Maskenbasiskörper nach Fig. 1a kompatiblen Dichtungsanordnung, die im wesentlichen im Bereich einer den Nasenrücken des Maskenanwenders überquerenden Dichtlippenauflagezone eine höhere Flächenpressung erzeugt;
**Fig. 1e:** eine Skizze zur Erläuterung des Verlaufs der Dichtlippenauflagezone einer Dichtungsanordnung gem. Fig. 1b;
**Fig. 1f:** eine Skizze zur Erläuterung des Verlaufs der Dichtlippenauflagezone bei einer Dichtkissenanordnung gem. Fig. 1c;
**Fig. 1g:** eine Skizze zur Erläuterung des Verlaufs der Dichtlippenauflagezone sowie der hierbei auftretenden Flächenpressungen bei Verwendung einer Dichtungsanordnung gem. Fig. 1d;
**Fig. 2a:** eine vereinfachte Seitenansicht einer Dichtungsanordnung mit integriertem Befestigungsrahmen sowie plastisch verformbarem Versteifungselement;
**Fig. 2b:** eine vereinfachte Schnittansicht durch eine Dichtlippeneinrichtung, wie sie in Fig. 2a Anwendung findet, mit integriertem, plastisch verformbarem Versteifungselement sowie Gelkörper;
**Fig. 2c, d, e, f:** Skizzen zur Erläuterung unterschiedlicher Verläufe des in die Dichtkisseneinrichtung nach Fig. 2b integrierten, plastisch verformbaren Versteifungselementes;

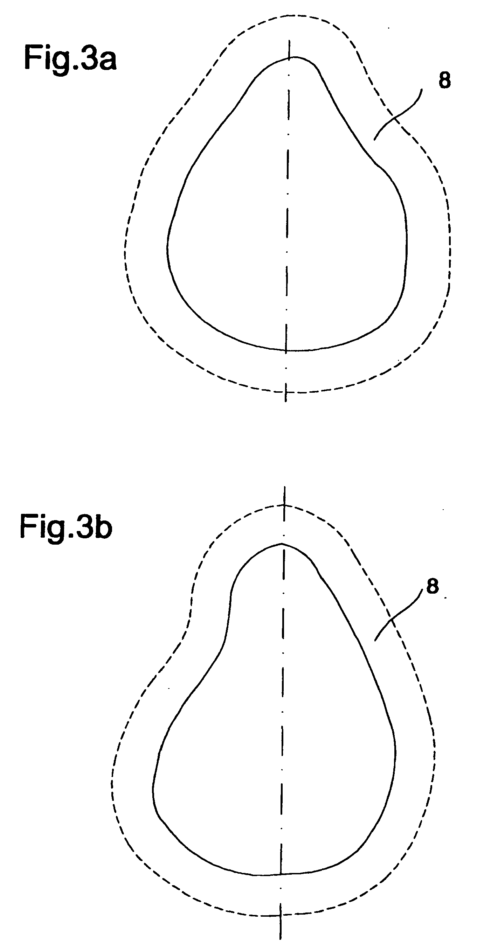

**Fig. 3a und 3b:** unterschiedliche Dichtlippenauflagezonen mit asymmetrischem Verlauf.

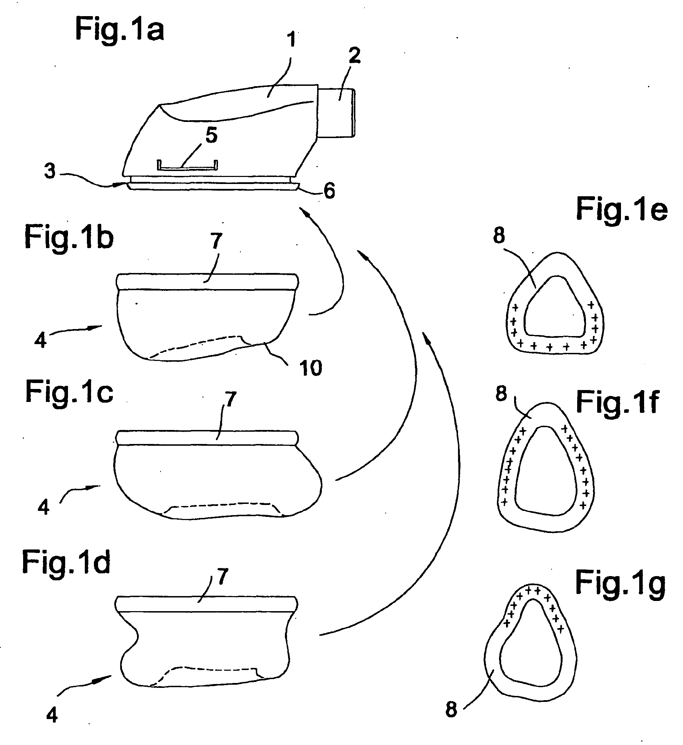

Der in Fig. 1a dargestellt, hier durch eine Hartschale 1 gebildete Maskenbasiskörper umfasst eine Schlauchanschlusseinrichtung 2 und eine Anschlussstruktur 3 zur Befestigung einer nachfolgend in Verbindung mit den Fig. 1b, 1c und 1d noch näher beschriebenen Dichtungsanordnung 4.

Die Hartschale 1 ist hier aus einem volltransparenten, thermoplastischen Kunststoffmaterial gebildet und mit zwei Haltebügeln 5 versehen, an welchen das untere Gurtbandpaar einer Kopfbandanordnung (nicht dargestellt) anbringbar ist.

Die Anschlussstruktur 3 weist hier einen Umfangswulst 6 auf, der mit einer entsprechend komplementär ausgebildeten Innenumfangsnut einer passenden Dichtungsanordnung in Eingriff bringbar ist. Durch den entsprechenden Eingriff von Umfangswulst und Innenumfangsnut wird eine lagerichtige und abdichtende Anbringung der Dichtungsanordnung an der Hartschale 1 gewährleistet. An der hier vorgesehenen Anschlussstruktur 3 sind die in den Fig. 1 b, 1 c und 1d dargestellten Dichtungsanordnungen 4 anbringbar.

Die in Fig. 1b dargestellt Dichtungsanordnung umfasst eine aus einem elastomeren Material gebildete Dichtlippeneinrichtung 3, die mit einem Rahmenabschnitt 7 versehen ist. Der Rahmenabschnitt 7 ist vergleichsweise dickwandig ausgebildet und trägt in seinem Innenbereich eine zur Anschlussstruktur 3 nach Fig. 1a komplementäre Befestigungsstruktur.

Die Dichtungsanordnungen 4 nach den Fig. 1c und 1d weisen ebenfalls Rahmenabschnitte 7 auf, die in gleicher Weise wie der Rahmenabschnitt 7 nach Fig. 1b komplementär zur Anschlussstruktur 3 nach Fig. 1a ausgebildet sind. Die in den Fig. 1 b, 1c und 1d dargestellten Dichtkisseneinrichtungen gewährleisten jeweils bei einer zulässigen Flächenpressungsverteilung bei ein und demselben Maskenanwender den gewünschten Abdichtungseffekt. Die unterschiedlichen Dichtungsanordnungen 4 unterscheiden sich ins besondere im Hinblick auf den durch sie bestimmten Verlauf der Auflagezone auf der Gesichtsfläche des Maskenanwenders.

Wie in Fig. 1e skizzenhaft dargestellt, wird beispielsweise durch die Dichtungsanordnung 4 gem. 1b eine vergleichsweise gedrungene Silhouette der Dichtlippenauflagezone 8 erreicht, wobei sich in dem die Oberlippe des Maskenanwenders überquerenden Bereich der Dichtlippenauflagezone 8 sowie in dem, den Nassenflügeln benachbarten Bereich vergleichsweise hohe Flächenpressungen ergeben.

Die in Fig: 1f dargestellt Dichtlippenauflagezone 8 wird durch eine Dichtkisseneinrichtung erreicht, wie sie in Fig. 1c dargestellt ist. Die Silhouette dieser Dichtlippenauflagezone ist gegenüber der in Fig. 1e skizzierten Dichtlippenauflagezone etwas länglicher ausgebildet. Bei dieser Dichtlippenauflagezone ergeben sich insbesondere im Bereich der Oberlippe sowie im Bereich des Nasenrückens geringere Flächenpressungen.

In Fig. 1d ist eine Dichtlippenauflagezone 8 dargestellt, wie sie beispielsweise durch die Dichtungsanordnung 4 gem. Fig. 1d erreicht wird. Bei dieser Dichtlippenauflagezone wird im Bereich des Nasenrückens eine vergleichsweise hohe Flächenpressung bewirkt, wogegen sich im Bereich der Nasenflügel sowie im Bereich der Oberlippe eine deutliche Entlastung ergibt.

Die hier vereinfacht dargestellten Dichtungsanordnungen nach Fig. 1 b, 1 c und 1d können beispielsweise im Abstand von 2 oder 3 Tagen zyklisch gewechselt werden, so dass sich im Abstand von 2 oder 3 Tagen auf dem Gesicht des Maskenanwenders unterschiedliche Flächenpressungsverteilungen ergeben. Hierdurch wird die Regeneration der stärker belasteten Gesichtsauflagezonen unterstützt.

Der Wechsel der Zonen höherer Flächenpressung, wie er bei den hier dargestellten Ausführungsformen erreicht wird, ist besonders vorteilhaft. Untersuchungen haben gezeigt, dass bereits durch geringe Änderung des Verlaufs lediglich der Auflagezöne eine ausreichende Regeneration des belasteten Gesichtsgewebes eintritt.

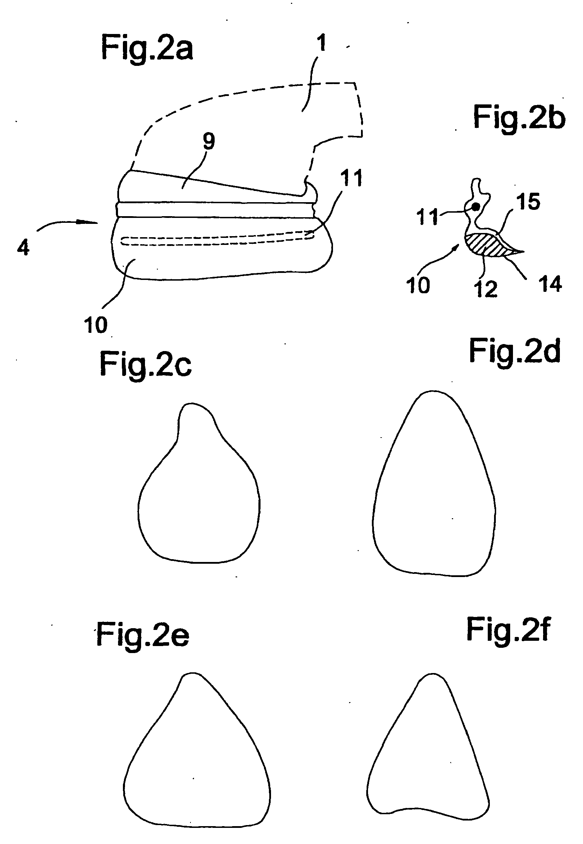

In Fig. 2a ist eine weitere Ausführungsform einer Dichtungsanordnung 4 dargestellt, die hier einen Schnellwechselrahmen 9, aufweist. Dieser Schnellwechselrahmen 9 ist über einen Schwenkrastmechanismus in abdichtender Weise mit einem Maskenbasiskörper 1 (hier nur andeutungsweise dargestellt) koppelbar. Der Schnellwechselrahmen 9 ist bei der hier dargestellten Ausführungsform aus einem Hartmaterial gebildet und trägt eine aus einem Elastomermaterial gebildete Dichtlippeneinrichtung 10. Die Dichtlippeneinrichtung 10 ist mit einem Versteifungselement 11 gekoppelt, das hier aus einem Metallwerkstoff gebildet ist. Durch das Versteifungselement 11 kann die Dichtlippeneinrichtung 10 willkürlich verformt und individuell an die Gesichtstektur des Maskenanwenders angepasst werden.

In Fig. 2b ist vereinfacht der Querschnitt durch eine besondere Ausführungsform der Dichtlippeneinrichtung 10 mit integriertem Versteifungselement 11 dargestellt.

Bei der hier dargestellten Ausführungsform umfasst die Dichtlippeneinrichtung einen aus einem Gelmaterial gebildeten Gelkörper 12, der zur Gesichtsauflagefläche hin mit einer elastomeren Haut 14 versehen ist.

Der Gelkörper 12 ist an einer Dichtlippe 15 angebracht, welche wiederum das Versteifungselement 11 aufnimmt. Durch Deformierung des Versteifungselementes 11 wird die Dichtlippe 15 und gemeinsam mit dieser der Gelkörper 12 in seinem Verlauf definiert festgelegt.

Derartige willkürliche Verläufe des Versteifungselementes 11 sind skizzenhaft in Fig. 2c, 2d, 2e und 2f dargestellt. Durch diese unterschiedlichen Konturen des Versteifungselementes 11 wird es möglich, die geforderte Dichtwirkung durch unterschiedliche Dichtlippenauflagezonen zu gewährleisten. Vorzugsweise werden vier in ihrem Aufbau gleichartige Dichtungsanordnungen 4 derart angepasst, dass die geforderte Dichtwirkung durch unterschiedliche Dichtlippenauflagezonen erreicht wird. Die derart individuell angeformten Dichtungsanordnungen 4 können beispielsweise im Abstand von zwei Tagen über den jeweiligen Rahmenabschnitt 9 unmittelbar an die Hartschale 1 angekoppelt werden, ohne dass weiterer Nachjustierungsbedarf besteht.

Gem. einer besonders bevorzugten Ausführungsform kann der Wechsel der Gesichtsauflagezone dadurch erreicht werden, dass zwei unterschiedliche Dichtungsanordnungen 4 verwendet werden, die asymmetrische Dichtlippenauflagezonen 8 bewirken, wobei diese asymmetrischen Dichtlippenauflagezonen 8 einen zueinander im wesentlichen spiegelsymmetrischen Verlauf aufweisen.

Die erfindungsgemäße Atemmaskenanordnung kann beispielsweise, wie nachfolgen beschrieben, Anwendung finden:

Zur Durchführung einer CPAP-Therapie zur Behandlung schlafbezogener Atmungsstörungen auf Grundlage einer pneumatischen Schienung der oberen Atemwege wird zunächst ein CPAP-Gerät bereitgestellt, welches über eine flexible Schlauchleitung mit einer Hartschale einer Maskenanordnung gekoppelt wird. An diese Hartschale wird aus einem Set von drei unterschiedlichen, jedoch für ein und denselben Maskenanwender passenden Dichtlippeneinrichtungen eine erste ausgewählt und mit der Hartschale 1 gekoppelt. Die so vorbereitete Atemmaskenanordnung kann nun über ein Kopfband auf dem Nasenbereich des Maskenanwenders fixiert werden. Der Patient kann nunmehr mit der Atemmaskenanordnung zu Bett gehen, wobei während des Schlafes über die Atemmaskenanordnung permanent Atemgas unter einem vorbestimmten Überdruck bereitgestellt ist. Nach Erwachen kann die Atemmaskenanordnung von der Schlauchleitung abgenommen und gereinigt werden. Hierbei wird die Dichtungsanordnung ausgetauscht, wobei nunmehr eine ebenfalls passende Dichtungsanordnung aus dem Set entnommen und an die Hartschale 1 angesetzt wird, die eine abweichende Gesichtsauflagezone verursacht. Nachdem auch mit dieser Dichtlippeneinrichtung eine Überdruckbeatmung während der Schlafphase des Maskenanwenders erfolgt ist, kann diese entweder mit der vorangehend verwendeten Dichtlippeneinrichtung oder einer weiteren Dichtlippeneinrichtung ausgetauscht werden. Durch die ständige Änderung der zur Anwendung kommenden Gesichtsauflagezone wird eine erhebliche Reduzierung der Belastung des Gesichtsgewebes des Maskenanwenders sowie eine verbesserte Regeneration der belasteten Gesichtszonen erreicht.

## Patentansprüche

1. Atemmaskenanordnung mit einem Maskenbasiskörper, einer Schlauchanschlusseinrichtung zum Anschluss eines Atemschlauches, einer Befestigungseinrichtung zur Fixierung des Maskenbasiskörpers im Nasenbereich eines Maskenanwenders und einer Dichtungsanordnung zur Abdichtung eines durch den Maskenbasiskörper begrenzten Maskeninnenraums gegenüber der Umgebung, wobei die Dichtungsanordnung eine aus einem elastomeren Material gebildete Dichtlippeneinrichtung aufweist, die entlang einer Gesichtsauflagezone unter einer definierten Flächenpressungsverteilung auf dem Gesicht des Maskenanwenders aufliegt, **dadurch gekennzeichnet, dass** die Dichtlippeneinrichtung aus einem Set aus wenigstens zwei unterschiedlichen Dichtlippeneinrichtungen ausgewählt ist, wobei die Dichtlippeneinrichtungen des Sets sich hinsichtlich der Flächenpressungsverteilung und/oder dem Verlauf der Gesichtsauflagezone unterscheiden und für den Maskenanwender jeweils passen, und wobei die Dichtlippeneinrichtungen hinsichtlich ihrer Dichtlippenauflagezonen einen asymmetrischen Verlauf aufweisen.

2. Atemmaskenanordnung nach Anspruch 1, **dadurch gekennzeichnet, dass** die Dichtlippeneinrichtungen Befestigungsstrukturen aufweisen, die zu einer maskenbasiskörperseitigen Befestigungsstruktur (3) kompatibel sind.

3. Atemmaskenanordnung nach Anspruch 1 oder 2, **dadurch gekennzeichnet, dass** das Dichtlippenset wenigstens drei unterschiedliche Dichtlippeneinrichtungen (10) aufweist.

4. Atemmaskenanordnung nach wenigstens einem der Ansprüche 1 bis 3, **dadurch gekennzeichnet, dass** die Dichtlippeneinrichtungen (10) durch unterschiedlich individuell angeformte Dichtlippeneinrichtungen (10) gebildet sind.

5. Atemmaskenanordnung nach wenigstens einem der Ansprüche 1 bis 4, **dadurch gekennzeichnet, dass** wenigstens eine der Dichtlippeneinrichtungen (10) einen Gelkörper (12) umfasst.

6. Atemmaskenanordnung nach wenigstens einem der Ansprüche 1 bis 5, **dadurch gekennzeichnet, dass** wenigstens eine der Dichtlippeneinrichtungen eine biegeverformbare oder unter Temperatureinwirkung plastisch verformbare Verstärkungseinlage (11) aufweist.

7. Atemmaskenanordnung nach wenigstens einem der Ansprüche 1 bis 6, **dadurch gekennzeichnet, dass** sich die Gesichtsauflagezone im Nasenbereich unterscheiden.

8. Atemmaskenanordnung nach wenigstens einem der Ansprüche 1 bis 7, **dadurch gekennzeichnet, dass** sich die Gesichtsauflagezonen (8) in dem den Nasenflügeln benachbarten Bereich hinsichtlich ihres Verlaufs und/oder hinsichtlich der Gesichtsflächenpressung unterscheiden.

9. Atemmaskenanordnung nach wenigstens einem der Ansprüche 1 bis 8, **dadurch gekennzeichnet, dass** jede Dichtlippeneinrichtung mit einem Rahmenelement (9) versehen ist, zum definierten Befestigen der Dichtlippeneinrichtung an dem Maskenbasiskörper (1).

10. Atemmaskenanordnung nach wenigstens einem der Ansprüche 1 bis 9, **dadurch gekennzeichnet, dass** jeder Dichtlippeneinrichtung ein Maskenbasiskörper zugeordnet ist, und die Dichtlippeneinrichtungen (10) mit dem Maskenbasiskörper (1) austauschbar sind.

11. Atemmaskenanordnung nach wenigstens einem der Ansprüche 1 bis 10, **dadurch gekennzeichnet, dass** sich die Dichtlippeneinrichtungen hinsichtlich der mit dem Gesicht zur Anlage gelangenden Oberflächenstruktur unterscheiden.

12. Atemmaskenanordnung nach wenigstens einem der Ansprüche 1 bis 11, **dadurch gekennzeichnet, dass** die Oberfläche einer Dichtlippeneinrichtung matt ist.

13. Atemmaskenanordnung nach wenigstens einem der Ansprüche 1 bis 12, **dadurch gekennzeichnet, dass** die Oberfläche einer Dichtlippeneinrichtung glattglänzend ist.

14. Atemmaskenanordnung nach wenigstens einem der Ansprüche 1 bis 13, **dadurch gekennzeichnet, dass** wenigstens eine der Dichtlippeneinrichtungen einen thermoverformbaren Rahmen aufweist.

## Claims

1. A breathing mask arrangement comprising a mask base body, a tube connection means for the connection of a breathing tube, a fixing means for fixing the mask base body in the nasal region of a mask user and a sealing arrangement for sealing a mask interior, which is limited by the mask base body, against the environment, wherein the sealing arrangement comprises a sealing lip means which is made of an elastomeric material and which lies on the face of the mask user along a face contact zone under a defined surface pressure distribution, **characterized in that** the sealing lip means is selected from a set of at least two different sealing lip means, wherein the sealing lip means of the set differ with respect to the surface pressure distribution and/or the course of the face contact zone and fit to the respective mask user, and wherein the sealing lip means have an asymmetric course with respect to their sealing lip contact zones.

2. The breathing mask arrangement according to claim 1, **characterized in that** the sealing lip means comprise fixing structures which are compatible with a fixing structure (3) on the side of the mask base body.

3. The breathing mask arrangement according to claim 1 or 2, **characterized in that** the set of sealing lips comprises at least three different sealing lip means (10).

4. The breathing mask arrangement according to at least one of claims 1 to 3, **characterized in that** the sealing lip means (10) are formed by different individually formed sealing lip means (10).

5. The breathing mask arrangement according to at least one of claims 1 to 4, **characterized in that** at least one of the sealing lip means (10) comprises a gel body (12).

6. The breathing mask arrangement according to at least one of claims 1 to 5, **characterized in that** at least one of the sealing lip means comprises a strengthening insert (11) that is deformable by bending or plastically deformable under the influence of temperature.

7. The breathing mask arrangement according to at least one of claims 1 to 6, **characterized in that** the face contact zones differ in the nasal region.

8. The breathing mask arrangement according to at least one of claims 1 to 7, **characterized in that** in the region close to the wings of the nose the face contact zones (8) differ from each other with respect to their course and/or with respect to the face surface pressure.

9. The breathing mask arrangement according to at least one of claims 1 to 8, **characterized in that** each sealing lip means comprises a frame element (9) for fixing the sealing lip means to the mask base body (1) in a defined manner.

10. The breathing mask arrangement according to at least one of claims 1 to 9, **characterized in that** a mask base body is assigned to each sealing lip means and the sealing lip means (10) can be exchanged with the mask base body (1).

11. The breathing mask arrangement according to at least one of claims 1 to 10, **characterized in that** the sealing lip means differ with respect to the surface structure contacting the face.

12. The breathing mask arrangement according to at least one of claims 1 to 11, **characterized in that** the surface of a sealing lip means is mat.

13. The breathing mask arrangement according to at least one of claims 1 to 12, **characterized in that** the surface of a sealing lip means is polished.

14. The breathing mask arrangement according to at least one of claims 1 to 13, **characterized in that** at least one of the sealing lip means comprises a thermo-deformable frame.

## Revendications

1. Arrangement de masque respiratoire comprenant un corps de base du masque, un dispositif de connexion pour la connexion d'un tuyau respiratoire, un dispositif de fixation pour la fixation du corps de base du masque dans la zone du nez d'un utilisateur et un arrangement de joint pour l'étanchement vis-à-vis l'environnement d'une espace intérieure du masque limitée par le corps de base du masque, la disposition de joint ayant un dispositif à lèvres d'étanchéité formé d'un matériau élastomère qui repose sur le visage de l'utilisateur du masque le long d'une zone de repos sous une distribution de pression de surface définie, **caractérisé en ce que** le dispositif à lèvres d'étanchéité est choisi parmi un kit d'au moins deux dispositifs à lèvres d'étanchéité différents, les dispositifs à lèvres d'étanchéité dudit kit se différant en ce qui concerne la distribution de pression de surface et/ou de l'allure de la zone de repos sur le visage et étant adaptable pour l'utilisateur respectif, et les dispositifs à lèvres d'étanchéité ayant une allure asymétrique en ce qui concerne leurs zones de repos.

2. Arrangement de masque respiratoire selon la revendication 1, **caractérisé en ce que** les dispositifs à lèvres d'étanchéité comprennent des structures de fixation étant compatibles avec une structure de fixation (3) de la part du corps de base du masque.

3. Arrangement de masque respiratoire selon la revendication 1 ou 2, **caractérisé en ce que** le kit de lèvres d'étanchéité comprend au moins trois dispositifs à lèvres d'étanchéité (10) différents.

4. Arrangement de masque respiratoire selon au moins l'une des revendications 1 à 3, **caractérisé en ce que** les dispositifs à lèvres d'étanchéité (10) sont formés par des dispositifs à lèvres d'étanchéité différents formés individuellement.

5. Arrangement de masque respiratoire selon au moins l'une des revendications 1 à 4, **caractérisé en ce que** au moins l'une des dispositifs à lèvres d'étanchéité (10) comprend un corps de gel (12).

6. Arrangement de masque respiratoire selon au moins l'une des revendications 1 à 5, **caractérisé en ce que** au moins l'une des dispositifs à lèvres d'étanchéité comprend une insertion de renforcement (11) déformable par pliage ou déformable plastiquement sous l'influence de température.

7. Arrangement de masque respiratoire selon au moins l'une des revendications 1 à 6, **caractérisé en ce que** les zones de repos sur le visage se diffèrent dans la zone du nez.

8. Arrangement de masque respiratoire selon au moins l'une des revendications 1 à 7, **caractérisé en ce que** les zones de repos sur le visage (8) se diffèrent dans la zone voisinée des ailes du nez en ce qui concerne leur allure et/ou en ce qui concerne la pression de surface sur le visage.

9. Arrangement de masque respiratoire selon au moins l'une des revendications 1 à 8, **caractérisé en ce que** chacun des dispositifs à lèvres d'étanchéité (10) est équipé d'un élément de cadre (9) pour la fixation définie du dispositif à lèvres d'étanchéité au corps de base du masque.

10. Arrangement de masque respiratoire selon au moins l'une des revendications 1 à 9, **caractérisé en ce que** un corps de base du masque est accordé à chacun des dispositifs à lèvres d'étanchéité, et les dispositifs à lèvres d'étanchéité (10) sont interchangeables avec le corps de base du masque (1).

11. Arrangement de masque respiratoire selon au moins l'une des revendications 1 à 10, **caractérisé en ce que** les dispositifs à lèvres d'étanchéité se diffèrent en ce qui concerne la structure superficiel entrant en contact avec le visage.

12. Arrangement de masque respiratoire selon au moins l'une des revendications 1 à 11, **caractérisé en ce que** la surface d'un dispositif à lèvres d'étanchéité est mate.

13. Arrangement de masque respiratoire selon au moins l'une des revendications 1 à 12, **caractérisé en ce que** la surface d'un dispositif à lèvres d'étanchéité est lisse.

14. Arrangement de masque respiratoire selon au moins l'une des revendications 1 à 13, au moins l'une des dispositifs à lèvres d'étanchéité comprend un cadre thermodéformable.
